Europäisches Patentamt

(19) European Patent Office

Office européen des brevets

(11) Veröffentlichungsnummer: **0 028 827**
**A2**

(12) **EUROPÄISCHE PATENTANMELDUNG**

(21) Anmeldenummer: 80106890.9

(22) Anmeldetag: 08.11.80

(51) Int. Cl.³: **G 01 N 33/32**
**G 01 N 11/04**

(30) Priorität: 09.11.79 DE 2945396

(43) Veröffentlichungstag der Anmeldung:
20.05.81 Patentblatt 81/20

(84) Benannte Vertragsstaaten:
AT CH DE FR GB IT LI

(71) Anmelder: Fraunhofer-Gesellschaft zur Förderung der angewandten Forschung e.V.
Leonrodstrasse 54
D-8000 München 19(DE)

(72) Erfinder: Kassenbeck, Paul
Marie-Curie-Strasse 3
D-7513 Stutensee(DE)

(54) Verfahren und Einrichtung zum Messen des Zügigkeitsverhaltens von viskosen Flüssigkeiten, insbesondere von Druckfarben.

(57) Neben der Viskosität ist für die Zügigkeit auch die dynamische Oberflächenspannung viskoser Flüssigkeiten, wie z.B. Druckfarben von ausschlaggebender Bedeutung. Es besteht eine direkte Beziehung zwischen Zügigkeitsverhalten (Fadenabrißlänge) und der Einengung des Flüssigkeitsstrahls beim freien Fließen aus einer Öffnung (Strahlprofil).

Das Meßverfahren beruht auf der Ermittlung der linearen Strömungsgeschwindigkeiten in verschiedenen Abständen von der Düse, aus dem die viskose Flüssigkeit als freier Strahl mit konstantem Mengenstrom austritt.

Die Differenz bzw. der Quotient der linearen Strömungsgeschwindigkeiten (Strahlquerschnitte) ist ein Maß für das Zügigkeitsverhalten.

Die Einrichtung zur Messung einer linearen Strömungsgeschwindigkeit $v_f$ mit einstellbarem Abstand x von der Düse besteht aus einem Förderband mit regelbarer Geschwindigkeit u, auf das der Flüssigkeitsstrahl abgelegt wird. Die Bandgeschwindigkeit ist gleich der gesuchten linearen Strömungsgeschwindigkeit ($v_f = u$), wenn beim Vermindern der Bandgeschwindigkeit erstmals ein Rückstau des Strahles am Förderband eintritt.

0028827

Fraunhofer-Gesellschaft zur Förderung der angewandten
Forschung e.V.
Leonrodstraße 54, 8000 München 19


Verfahren und Einrichtung zum Messen
des Zügigkeitsverhaltens von viskosen
Flüssigkeiten, insbesondere von Druckfarben

Die Erfindung betrifft ein Verfahren und eine Einrichtung zum Messen des Zügigkeitsverhaltens von viskosen
Flüssigkeiten, insbesondere von Druckfarben.

Druckfarben, wie sie für das Bedrucken textiler Flächengebilde im Rouleaux- und Rotationsfilmdruck eingesetzt
werden, enthalten neben dem Farbstoff auch Chemikalien
und mindestens ein Verdickungsmittel (meist hochmolekulare Polysaccharide pflanzlicher Herkunft) in wässeriger
Lösung. Die Menge der genannten und in Lösung befindlichen Substanzen bildet den sogenannten Festkörpergehalt,
der in Gewichtsprozenten angegeben wird (FG %). Es gibt
"körperarme" und "körperreiche" Verdickungen je nach dem
prozentualen Anteil an Verdickungsmitteln, der erforderlich ist, um eine Viskosität zu erzielen, die einen guten
Druckausfall hinsichtlich der Farbstoffaufnahme und der
Druckschärfe unter gegebenen Bedingungen auf der Druckmaschine garantieren. Neben dem Viskositätsverhalten ist
für den Textildrucker auch die Zügigkeit einer Druckfarbe von Bedeutung. Unter diesem Begriff versteht man die
ligenschaft eines Flüssigkeitsstrahles beim freien Fließen von einem Stab oder aus einer Öffnung vor dem Abriß
einen mehr oder weniger langen Faden zu bilden. Zügige
Druckfarben, die also längere Fäden bilden, ergeben meist
einen besseren Druckausfall. Es gibt jedoch Ausnahmen zu
dieser rein empirisch aufgestellten Regel. Neben der Viskosität ist für die Zügigkeit auch die dynamische Oberflächenspannung von ausschlaggebender Bedeutung. Es besteht eine direkte Beziehung zwischen Zügigkeitsverhalten (Fadenabrißlänge) und der Einengung des Flüssigkeitsstrahles beim freien Fließen aus einer Öffnung (Strahlprofil). Je enger die Einschnürung ist, desto früher kommt es zum Abriß und desto geringer erscheint die Zügigkeit bei gleicher Fördermenge. Um die Druckfarbe vom rheologischen Verhalten her optimal einstellen zu können, ist es für den Textildrucker wichtig, deren Zügigkeitsverlauf über einen möglichst breiten Bereich des Festkörpergehaltes (Viskosität) zu kennen.

Bisher erfolgte die Bestimmung des Zügigkeitsverhaltens von viskosen Flüssigkeiten, insbesondere von Druckfarben, rein empirisch, indem in die zu untersuchende Flüssigkeit ein Stab getaucht und soweit herausgezogen wurde, bis der ablaufende Flüssigkeitsfaden abriß. Die Fadenlänge wurde dabei durch das Auge des Messenden näherungsweise bestimmt bzw. durch die Unterbrechung eines elektrischen Kontaktes ermittelt, wobei der Flüssigkeitsfaden als Stromleiter dient. Diese Verfahren sind ungenau und mit individuellen Meßfehlern behaftet.

Aufgabe der Erfindung ist es deshalb, ein automatisch durchführbares Verfahren und eine Meßeinrichtung anzugeben, die eine einwandfreie und reproduzierbare Bestimmung des Zügigkeitsverhaltens von viskosen Flüssigkeiten ermöglicht. Ein erfindungsgemäßes Verfahren zur Lösung dieser Aufgabe ist im Patentanspruch 1 angegeben. Weiterbildungen dieses Verfahrens enthalten die Ansprüche 2 und 3, während die Ansprüche 4 bis 6 auf eine zur Durchführung des Verfahrens geeignete Meßeinrichtung betreffen.

Die Erfindung beruht auf der Erkenntnis, daß das Zügigkeitsverhalten nicht nur durch die einer Messung schwer zugänglichen Länge eines Flüssigkeitsfadens, sondern auch durch das Einschnürungsverhalten eines frei fallenden Flüssigkeitsstrahles bestimmt werden kann, wobei die Bestimmung der Strahlquerschnitte durch Messungen der Strahlgeschwindigkeiten bei vorgegebenen Fallhöhen ersetzt werden kann.

Im folgenden ist die Erfindung beispielsweise anhand der Zeichnungen beschrieben. Es zeigen:

Figur 1    eine erfindungsgemäße Meßeinrichtung schematisch,

Figur 2    einen vergrößerten Ausschnitt aus der Figur 1 mit einer schematischen Darstellung des Fließkegels, des Förderbandes und des auf dem Förderband abgelegten Flüssigkeitsfadens

Figur 3    eine Photomontage verschiedener Fadenspuren,

Figuren 4-6    mit einer erfindungsgemäßen Meßeinrichtung aufgenommene Zügigkeitskurven

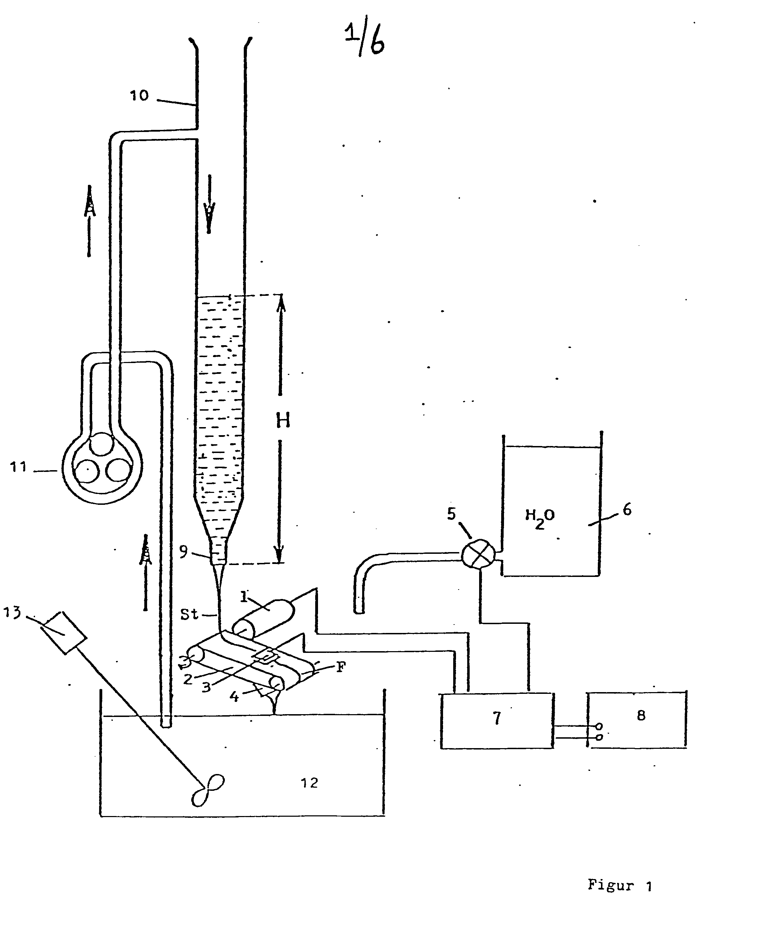

Die in Figur 1 schematisch dargestellte Einrichtung besteht aus einem Standrohr 10 von 30 mm Durchmesser und 70 cm Höhe, in welches über eine Pumpe 11 mit regelbarer Förderleistung die zu untersuchende Flüssigkeit aus dem Vorratsbehälter 12 gepumpt wird. Das vertikale Standrohr ist am unteren Ende mit einer Austrittsdüse 9 von 3 mm Durchmesser versehen. Bei gegebener Förderleistung der Pumpe stellt sich im Standrohr 10 ein Flüssigkeitsniveau mit der Standhöhe H über der Düsenöffnung ein. Der aus der Düse 9 austretende Flüssigkeitsstrahl St wird auf ein transparentes Förderband 2 abgelegt, dessen Geschwindigkeit während der Messung über einen Motor 1 von einem Steuergerät 7 geregelt wird. Der auf dem Förderband 2 abgelegte Flüssigkeitsfaden F wird durch eine Lichtschranke 3 geleitet, deren Funktion es ist, über ein Relais-System die Geschwindigkeit des Antriebsmotors 1 zu steuern. Die Flüssigkeit des Fadens F gelangt dann über eine Rakel 4 wieder in den Vorratsbehälter 12 zurück. Aus einem zweiten Behälter 6 für Wasser wird die Verdünnungsflüssigkeit nach Bedarf über ein Dosierventil 5 verdünnt. Ein Rührwerk 13 sorgt für die rasche Durchmischung des zugeleiteten Wassers bzw. Verdünnungsmittels.

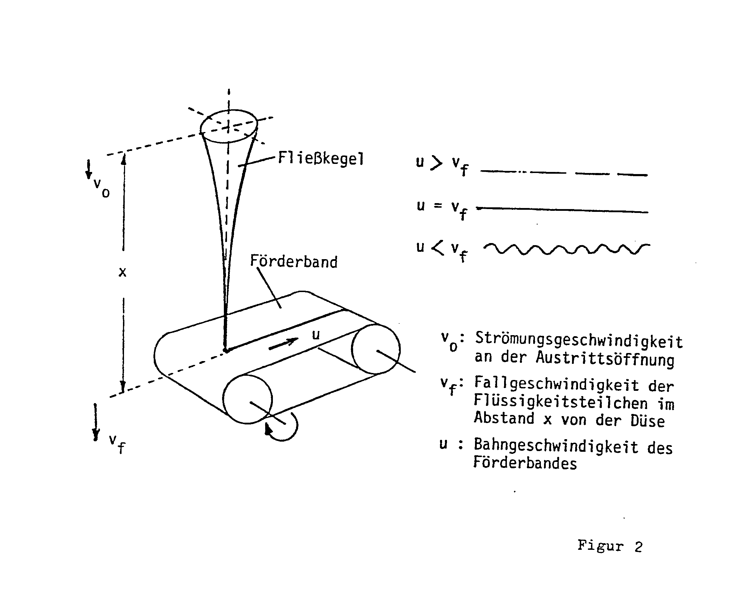

Die beschriebene Einrichtung gestattet eine genaue und vollautomatische Messung der linearen Strömungsgeschwindigkeit $v_f$ der Flüssigkeitsteilchen im Strahl in einem vorgegebenen Abstand x von der Austrittsdüse 9 (Figur 2). Wird die Umlaufgeschwindigkeit des Förderbandes mit u bezeichnet, so sind folgende Situationen möglich:

1. $u > v_f$ Der Flüssigkeitsstrahl wird auf dem Förderband verstreckt; sein Profilbild ist gradlinig. Je nach Geschwindigkeit und rheologischem Verhalten der Flüssigkeit kann es hierbei zum Abriß des Fadens kommen, namentlich wenn $u \gg v_f$.

2. $u = v_f$ Der Strahl wird geradlinig abgelegt und passiert ohne jede Beeinflussung die Lichtschranke wie in Punkt 1.

3. $u < v_f$ Durch den spontan einsetzenden Stau wird der Strahl in einer periodischen Schwingung seitlich abgelenkt und dementsprechend in Form einer Wellenlinie auf dem Förderband abgelegt. Die Lichtschranke wird hierbei durch den Flüssigkeitsfaden verdeckt und ein Signal ausgelöst, welches zur Steuerung des Meßvorganges dient. (Bei Elektrolyten kann statt der Lichtschranke auch ein aus beispielsweise feinen Platindrähten bestehender Kontaktgeber verwendet werden).

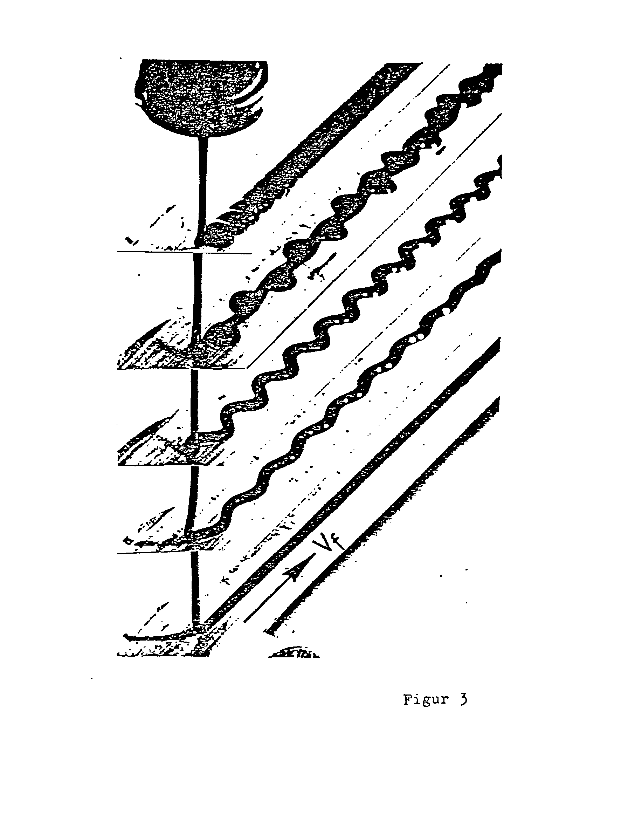

Die Situationen 2 und 3 sind in Figur 3 in einer Fotomontage veranschaulicht:
im unteren Bild sind Fließgeschwindigkeit und Förderbandgeschwindigkeit gleich; der Strahl wird geradlinig abgelegt. Die darüber liegenden 4 Aufnahmen zeigen von unten nach oben das Profil des Strahles auf dem Förderband bei progressiver Verringerung der Umlaufgeschwindigkeit (u).

Aus der Forderung, daß durch jeden Querschnitt des Fließkegels in der Zeiteinheit die gleiche Flüssigkeitsmenge strömen muß (Kontinuitätsprinzip), ergibt sich für den Durchmesser (d) des Fließkegels im Abstand x von der Austrittsdüse:

$$d = 2\sqrt{\frac{R^2 \cdot v_o}{v_f}}$$

(R: Radius der Austrittsdüse, $v_o$ : ergibt sich aus der Fördermenge $V/R^2\pi \cdot t = h/t = v_o$), wobei V das Volumen der in der Zeiteinheit durch die Austrittsöffnung strömenden Flüssigkeit ist und h die Höhe des zugehörigen Flüssigkeitszylinders der Grundfläche $R^2\pi$).
Unter sonst gleichbleibenden Bedingungen ist der Durchmesser des Strahles im Abstand x von der Düse lediglich von der Oberflächenspannung und der Viskosität der Flüssigkeit abhängig. Bei Zu- oder Abnahme eines bzw. beider dieser genannten Faktoren wird der Strahldurchmesser im Abstand x gleichsinnig beeinflußt und nimmt ebenfalls zu oder ab. Der Grund liegt darin, daß sowohl die durch molekulare Kohäsionskräfte verursachte innere Reibung (Viskosität) als auch die zur Vergrößerung der Strahloberfläche erforderliche Arbeit (Oberflächenspannung) Energie verbrauchen, welche nur der kinetischen Energie der fallenden Flüssigkeitsteilchen entnommen werden kann. Die mit zunehmender Fallgeschwindigkeit einhergehende Einengung des Strahles bewirkt gleichzeitig eine Expansion seiner Oberfläche, da mit zunehmendem Abstand von der Austrittsdüse das Verhältnis Oberfläche zu Volumen in Strahlabschnitten gleicher Länge größer wird. Die Einengung des Strahles ist deshalb um so geringer, je höher die Oberflächenspannung ist.

Mit der beschriebenen Meßeinrichtung können "Zügigkeitskurven" automatisch auf einem Schreiber 8 registriert werden, wobei hier anstelle der einer Messung schwer zugänglichen Fadenlänge die mit der Zügigkeit korrelierte Änderung des Strahlquerschnittes registriert wird. Voraussetzung ist, daß innerhalb einer Meßreihe die Strömungsgeschwindigkeit als Parameter an der Austrittsdüse konstant gehalten wird. Die zu prüfende Druckfarbe wird zunächst hochviskos angesetzt und in den Vorratsbehälter 12 eingefüllt. Die Förderleistung der Pumpe 11 wird so geregelt, daß sich im Standrohr 10 eine Standhöhe H ergibt, die einer Fördermenge von etwa 6 bis 20 ml/min entspricht. Sobald der Strahl St aus der Düse 9 fließt, wird über das Steuergerät 7 der Antriebsmotor 1 des Förderbandes 2 eingeschaltet und auf eine Geschwindigkeit $u > v_f$ gebracht. Wenn das Niveau im Standrohr 10 stabilisiert ist, (etwa nach 15 bis 30 Minuten, je nach Viskosität der Flüssigkeit und Förderleistung der Pumpe) kann der eigentliche, vom Steuergerät 7 aus programmierte Meßvorgang beginnen. Die Geschwindigkeit des Förderbandes 2 wird automatisch verringert bis der erste Stau im Flüssigkeitsstrahl auftritt. Das von der Lichtschranke 3 ausgelöste Signal betätigt ein Relais, über welches die zugehörige Bahngeschwindigkeit ($u_1 = v_{f1}$) des Förderbandes gespeichert wird. Dieser Vorgang wird im Meßabstand $x + \Delta x$ von der Düse 9 wiederholt für $u_2 = v_{f2}$. Die Differenz bzw. der

Quotient beider Werte $u_1$, $u_2$ wird auf dem Schreiber 8 registriert. Der Motor ($M_1$) wird anschließend wieder auf seine Ausgangsgeschwindigkeit gebracht (zwischen 10 und 15 cm/s ).

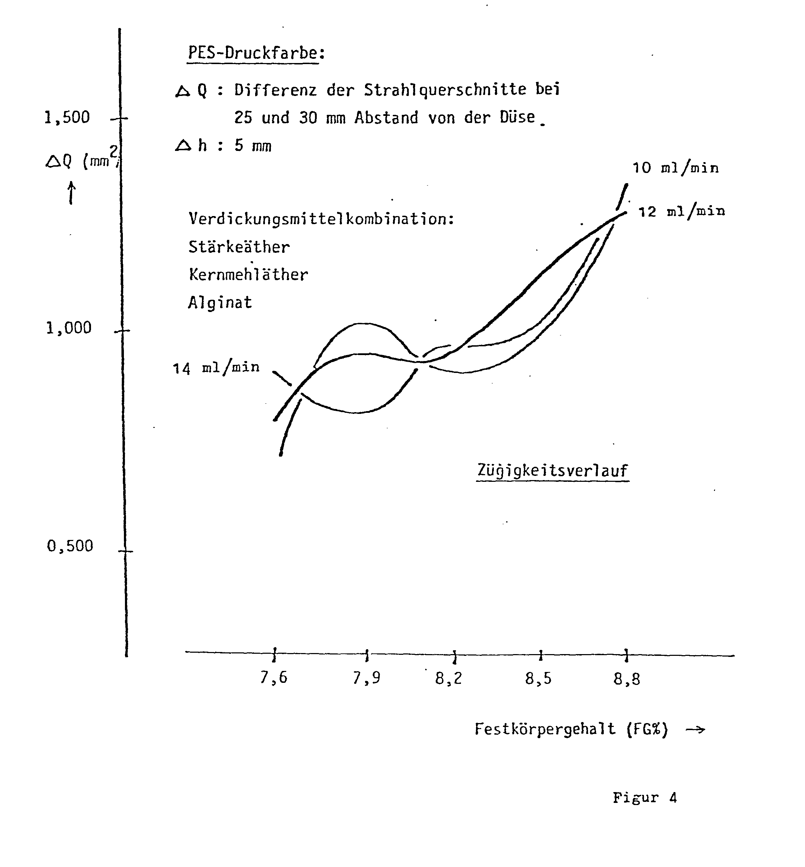

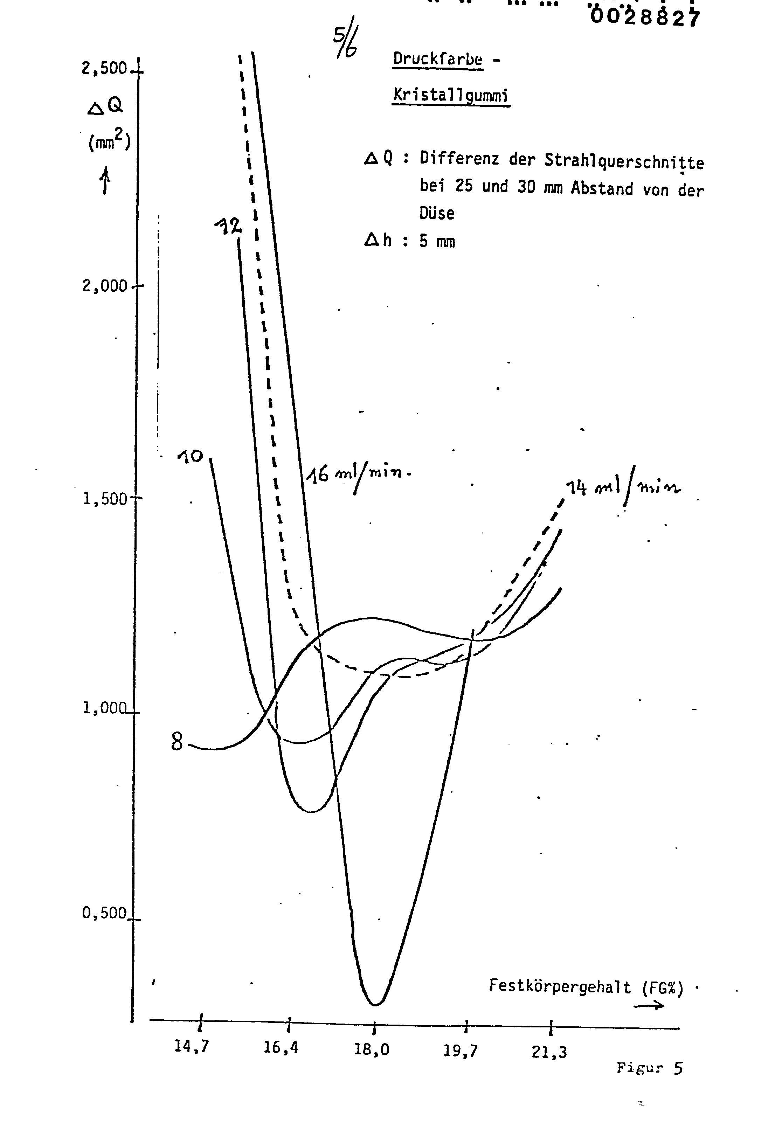

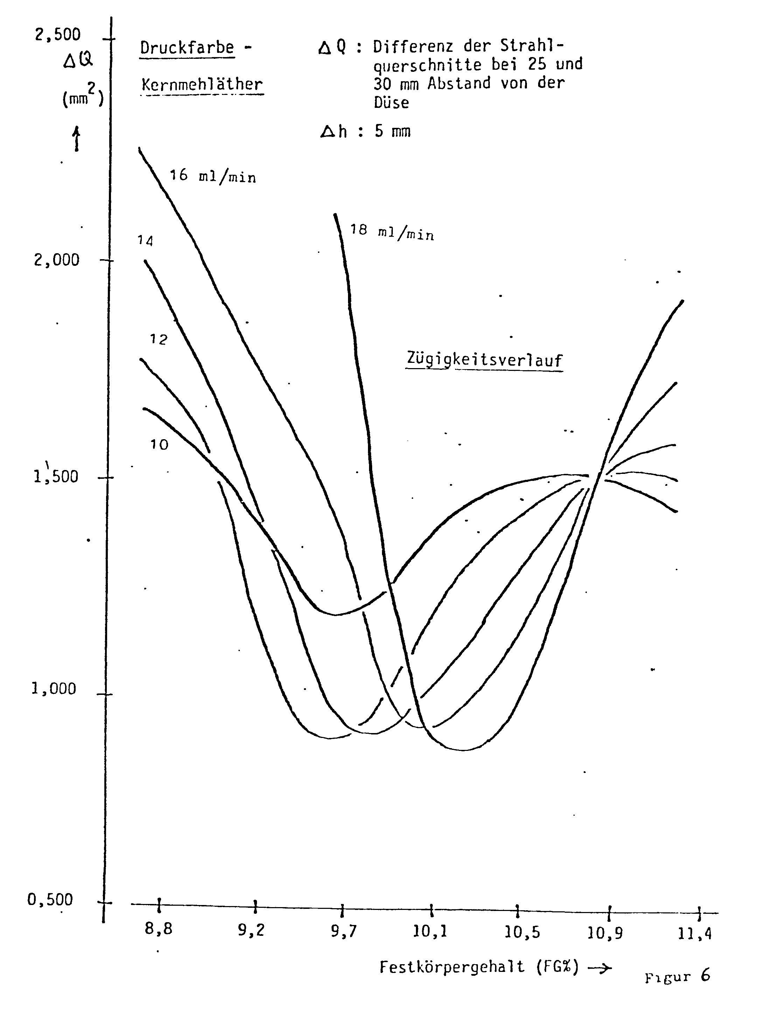

Ein Schalter betätigt nun das Rührwerk 13 und öffnet das Dosierventil 5, über welches etwa 50 ml Wasser/1 Druckfarbe in den Vorratsbehälter 12 gelangen. Nach etwa 20 min Rührzeit kann der Meßvorgang von neuem beginnen und ein weiterer Punkt der Zügigkeitskurve registriert werden. Als Ergebnis erhält man die in den Figuren 4, 5 und 6 veranschaulichten Zügigkeitskurven, die je nach der Art der Verdickungsmittel und der Fördermenge ein oder mehrere Minima aufweisen können, wobei das Minimum der Querschnittsänderung dem Zügigkeitsmaximum entspricht. Aus diesem Kurvenlauf kann der Textildrucker entnehmen, welche Bereiche des Festkörpergehaltes je nach Art der eingesetzten Verdickungsmittel zu meiden sind, um einen gleichmäßigen Druck zu erzielen bzw. welchen Einfluß unterschiedliche Farbstoffe, Stell- und Verdickungsmittel auf den Zügigkeitsverlauf und damit auch auf den Druckausfall haben.

Weitere Anwendungsgebiete für dieses Gerät und die beschriebene Meßmethode dürften in der Farben-, Lack- und Lebensmittelindustrie zu finden sein. Das Gerät kann seinem Prinzip nach in der Industrie nicht nur für Prüf- bzw. Kontrollzwecke, sondern auch als Steuerungsorgan eingesetzt werden, beispielsweise zur automatischen Regelung der Strömungsgeschwindigkeit einer Flüssigkeit.

Zur Berechnung der Querschnittsdifferenz bzw. der Querschnittseinengung im Strahl anhand der Meßwerte und der Gerätekonstanten dienen die folgenden Gleichungen mit einer Tabelle :

f : Durchflußmenge (ml/min) an der Austrittsdüse vom Radius $R_O = 0,15$ cm und der Querschnittsfläche

$$F = R_O^2 . \pi = 7,068 . 10^{-2} \ (cm^2)$$

$v_0$: Lineare Strömungsgeschwindigkeit der Flüssigkeitsteilchen an der Austrittsdüse

| f(ml/min) | $f(cm^3 . s^{-1})$ | | $v_O = f/R_O^2 . \pi \ (cm \cdot s^{-1})$ |
|---|---|---|---|
| 4 | 6,6 | $10^{-2}$ | 0,9432 |
| 6 | 1,0 | $10^{-1}$ | 1,4148 |
| 8 | 1,3 | $10^{-1}$ | 1,8864 |
| 10 | 1,6 | $10^{-1}$ | 2,3580 |
| 12 | 2,0 | $10^{-1}$ | 2,8296 |
| 14 | 2,3 | $10^{-1}$ | 3,3012 |
| 16 | 2,6 | $10^{-1}$ | 3,7728 |
| 18 | 3,0 | $10^{-1}$ | 4,2445 |
| 20 | 3,3 | $10^{-1}$ | 4,7161 |

$v_f$ : gemessene Strömungsgeschwindigkeit im Abstand h von der Düse $R_O^2 . v_a = r^2 . v_f$

daher

$$r = R_O \sqrt{\frac{v_O}{v_f}} = 0,15 \sqrt{\frac{v_O}{v_f}} \ (cm)$$

Q : Strahlquerschnitt im Abstand h von der Austrittsdüse

$$r^2 . \pi = 7,0686 . v_O/v_f \ (mm^2)$$

$\Delta Q : Q_1 - Q_2$

$Q_1/Q_2$ : Strahleinengung im Bereich $\Delta h$ gemessen bei den Düsenabständen $h_1$ und $h_2$

$$Q_1/Q_2 = v_{f2}/v_{f1}$$

- 1 -

Patentansprüche :

1. Verfahren zum Messen des Zügigkeitsverhaltens von viskosen Flüssigkeiten, insbesondere von Druckfarben, dadurch gekennzeichnet, daß die Flüssigkeit mit konstanter Fließgeschwindigkeit in ein Standrohr gefördert wird und durch eine am Boden des Standrohres angeordnete Düse als freier Strahl austritt, daß an dem Strahl in verschiedenen Abständen von der Düse dessen lineare Strömungsgeschwindigkeiten ermittelt und aus dem Quotienten bzw. der Differenz der Strömungsgeschwindigkeiten bzw. Strahlquerschnitte auf das Zügigkeitsverhalten der Flüssigkeit geschlossen wird.

2. Verfahren nach Anspruch 1,
dadurch gekennzeichnet,
daß die Strahlquerschnitte durch Messung der konstanten Flüssigkeitszufuhr zu dem Standrohr und der Strahlgeschwindigkeit an den Meßpunkten bestimmt wird.

3. Verfahren nach Anspruch 1 oder 2,
dadurch gekennzeichnet,
daß der Flüssigkeitsstrahl zur Messung seiner Geschwindigkeit auf ein im gewünschten Abstand von der Düse angeordnetes, mit verminderbarer Geschwindigkeit laufendes Förderband trifft und die Bandgeschwindigkeit ermittelt wird, bei der erstmals ein Rückstau des Strahles am Förderband eintritt.

4. Meßeinrichtung zur Durchführung des Verfahrens nach Anspruch 1 bis 3
gekennzeichnet durch

a) ein Standrohr (10), das durch eine Pumpe (11) mit regelbarer Förderleistung aus einem Vorratsgefäß (12) mit der zu untersuchenden Flüssigkeit gespeist werden kann und an seinem Boden eine Ausströmdüse (9) aufweist,

b) ein in veränderbarem Abstand (x) unter der Düse (3) angeordnetes Förderband (2) mit einem in seiner Drehzahl regelbaren Antriebsmotor (1) und einer den Bereich des von dem auf das Förderband (2) aufgelaufenen Flüssigkeitsstrahl (st) gebildeten Flüssigkeitsfaden (F) überwachenden Lichtschranke (3),

c) ein Steuergerät (6), das die Geschwindigkeit des Förderbandes (2) von einem vorgegebenen Höchstwert aus stetig vermindert bis die Lichtschranke (3) durch Verbreiterung des Flüssigkeitsfadenbereiches ausgelöst wird,

d) ein Registriergerät (7), das die Bandgeschwindigkeit im Auslösezeitpunkt der Lichtschranke (3) aufzeichnet.

5. Meßeinrichtung nach Anspruch 4,
dadurch gekennzeichnet,
daß ein Behälter (8) für eine Verdünnungsflüssigkeit, vorzugsweise Wasser, vorgesehen ist, deren Zufuhr zu dem mit einem Rührwerk (13) versehenen Vorratsgefäß (12) über ein Dosierventil (5) steuerbar ist.

- 3 -

6. Meßeinrichtung nach Anspruch 4 oder 5,
   dadurch gekennzeichnet,
   daß der Vorratsbehälter (12) unterhalb des Förderbandes (2) angeordnet ist, an dem sich eine Rakel (4) zum Abstreifen des Flüssigkeitsfadens (F) befindet.

7. Meßeinrichtung nach Anspruch 4,
   dadurch gekennzeichnet,
   daß bei stromleitenden Flüssigkeiten die Lichtschranke durch einen elektrischen Kontaktgeber ersetzt wird.

0028827

Figur 1

2|6

Figur 2

Figur 3

4/6

PES-Druckfarbe:

$\triangle$ Q : Differenz der Strahlquerschnitte bei
25 und 30 mm Abstand von der Düse.

$\triangle$ h : 5 mm

Verdickungsmittelkombination:
Stärkeäther
Kernmehläther
Alginat

$\triangle$Q (mm²)

14 ml/min

10 ml/min

12 ml/min

Zügigkeitsverlauf

1,500

1,000

0,500

7,6    7,9    8,2    8,5    8,8

Festkörpergehalt (FG%) →

Figur 4

5/6

Druckfarbe -

Kristallgummi

ΔQ : Differenz der Strahlquerschnitte
bei 25 und 30 mm Abstand von der
Düse

Δh : 5 mm

Figur 5

Figur 6